Europäisches Patentamt

(19) European Patent Office

Office européen des brevets

(11) Veröffentlichungsnummer: **0 004 566**
**B1**

# (12) EUROPÄISCHE PATENTSCHRIFT

(45) Veröffentlichungstag der Patentschrift:
**10.02.82**

(21) Anmeldenummer: **79100687.7**

(22) Anmeldetag: **08.03.79**

(51) Int. Cl.³: **A 61 B 3/12**

(54) **Vorrichtung zur Abbildung von Optotypen.**

(30) Priorität: **07.04.78 DE 2815121**

(43) Veröffentlichungstag der Anmeldung:
**17.10.79 Patentblatt 79/21**

(45) Bekanntmachung des Hinweises auf die Patenterteilung:
**10.02.82 Patentblatt 82/6**

(84) Benannte Vertragsstaaten:
**DE FR GB IT**

(56) Entgegenhaltungen:
**DE-A-1 955 859**
**DE-B-1 016 462**
**US-A-3 524 702**
**US-A-3 536 383**

(73) Patentinhaber: **Herbert Schwind GmbH & Co. KG,**
**Goldbacher Strasse 57, D-8750 Aschaffenburg (DE)**

(72) Erfinder: **Reiner, Josef, Dr., Stefan-Lochner-Strasse 14,**
**D-5000 Köln 50 (DE)**

(74) Vertreter: **Grams, Klaus Dieter, Dipl.-Ing. et al,**
**Patentanwälte Jaeger, Grams & Pontani Kreuzweg 34,**
**D-8031 Stockdorf (DE)**

Vorrichtung zur Abbildung von Optotypen

Die Erfindung bezieht sich auf eine Vorrichtung zur Darstellung von Optotypen bei der subjektiven Prüfung von Fehlsichtigkeit.

Es ist bekannt, von einem Probanden subjektiv zu beurteilende Optotypen auf Papptafeln oder von der Rückseite her beleuchteten Glasfeldern an der Wand eines Refraktionsraumes sichtbar zu machen oder mittels eines Diaprojektors auf diese Wand zu projizieren. Der Proband betrachtet diese Optotypen bei der subjektiven Refraktion durch in einer Probierbrille eingesteckte oder in einem Phoropter eingeschaltete Korrektionsgläser. In diesem Fall ist also eine Papptafel an der Wand oder ein Beleuchtungskasten für auswechselbare Schriftzeichenträger oder ein Projektionsschirm für die Abbildung mit Hilfe eines Diaprojektors erforderlich.

Ferner ist es bekannt (US-A-3 524 702), die Optotypen auf die Netzhaut des zu prüfenden Auges mittels eines Kollimators über einen Teilerspiegel abzubilden. Dies geschieht mittels einer Vorrichtung, die auch der objektiven Prüfung von Fehlsichtigkeit dient und ein Gehäuse, einen im Gehäuse angeordneten Kollimator, Optotypen, die im Gehäuse in der dingseitigen Brennebene des Kollimators angeordnet sind, sowie einen Teilerspiegel im bildseitigen Strahlengang des Kollimators aufweist. Bei dieser bekannten Vorrichtung muss der Proband bei der subjektiven Prüfung von Fehlsichtigkeit in das Vorrichtungsgehäuse hineinsehen, wobei er hinter dem Teilerspiegel Vorrichtungsteile sieht, sofern dieser Bereich nicht vollständig abgedunkelt ist. Obwohl in diesem Fall zur Darstellung der Optotypen weder eine Papptafel, noch ein Beleuchtungskasten, noch ein Projektionsschirm notwendig sind, besteht doch der Nachteil, dass der Proband durch Gehäuse- und Vorrichtungsteile in seinem Blickfeld irritiert wird und dass das Auge akkomodiert, was zu Fehlern bei der subjektiven Prüfung der Fehlsichtigkeit führt. Der Erfindung liegt die Aufgabe zugrunde, die gattungsgemässe Vorrichtung derart auszubilden, dass die Optotypen so dargestellt werden, dass die subjektive Augenrefraktionsbestimmung nicht behindert wird.

Diese Aufgabe wird erfindungsgemäss dadurch gelöst, dass der Teilerspiegel ausserhalb des Gehäuses und so angeordnet ist, dass das zu prüfende Auge am Gehäuse vorbei durch den Teilerspiegel in den freien Raum sieht, und dass das Gehäuse eine Öffnung für den bildseitigen Strahlengang vom Kollimator zum Teilerspiegel aufweist.

Die Abbildung von Testzeichen mittels eines Kollimators und eines Teilerspiegels auf die Netzhaut des zu prüfenden Auges ist an sich bei der objektiven Refraktionsbestimmung bekannt (DE-A-1 955 859). Dabei sieht jedoch das Auge nicht in den freien Raum, sondern auf eine Sehprobentafel. Ferner ist es bei der objektiven Refraktionsbestimmung bekannt, das Auge durch einen Teilerspiegel blicken zu lassen (US-A-

3 536 383). In Blickrichtung hinter dem Teilerspiegel sind dabei jedoch eine Linse und eine Marke angebracht, so dass das Auge nicht in den freien Raum sieht.

Vorteilhafte Ausbildungen der Erfindung sind in den Unteransprüchen gekannzeichnet.

Die einzige Figur zeigt einen Schnitt durch ein Ausführungsbeispiel einer erfindungsgemässen Vorrichtung zur Darstellung von Optotypen bei der subjektiven Prüfung von Fehlsichtigkeit.

Die Vorrichtung weist in einem Gehäuse 8 einen Kollimator 2 auf, der aus einem Hohlspiegel 3 besteht, in dessen durch den Brennpunkt 4 gehender Brennebene Optotypen 1 angeordnet sind. Bei den Optotypen kann es sich um beliebige beleuchtete Optotypen handeln, beispielsweise um ein von der Rückseite her mittels einer geeigneten Beleuchtungsvorrichtung beleuchtetes Diapositiv. Die Optotypen 1 werden über einen unter 45° zur Ebene der Optotypen und zur optischen Achse des Hohlspiegels 3 geneigten halbdurchlässigen Teilerspiegel 5 zu dem Hohlspiegel hin abgebildet und von diesem durch den Teilerspiegel 5 hindurch durch eine Öffnung 14 im Gehäuse 8 in das Unendliche abgebildet. In den von dem Hohlspiegel kommenden Parallelstrahlengang ist ein zu der optischen Achse des Hohlspiegels um 45° geneigt angeordneter erster Teilerspiegel 6 vorgesehen, der mit Hilfe einer Halterung 7 an dem Gehäuse befestigt ist. Dadurch wird der aus dem Gehäuse vertikal austretende Parallelstrahlengang in einen horizontal verlaufenden Strahlengang umgelenkt.

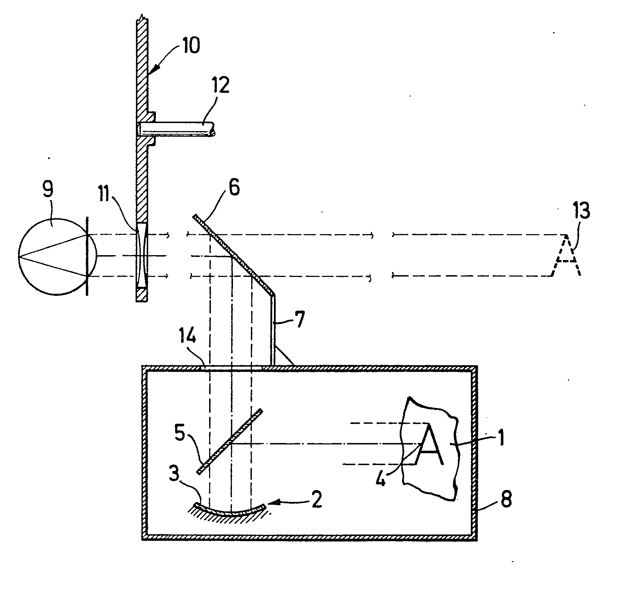

Die Vorrichtung wird zur Refraktion so in den Refraktionsraum aufgestellt, dass das zu untersuchende Auge 9 des Probanden einerseits durch die vorgeschaltete Korrektionslinse 11 und anderseits in den von dem ersten Teilerspiegel 6 kommenden Strahlengang schaut. Die Korrektionslinse 11 kann in einer Probierbrille oder wie in dem gezeigten Ausführungsbeispiel in einem Phoropter, der um eine Welle 12 drehbar ist, angeordnet sein. Nach Einstellen der der Fehlsichtigkeit entsprechenden Korrektionslinse 11 werden die von dem Hohlspiegel 3 kommenden Parallelstrahlen auf der Netzhaut des Auges 9 abgebildet, so dass der Proband den Eindruck hat, dass er das Bild 13 der Optotypen durch den Teilerspiegel 6 hindurch im freien Raum sieht.

Der Hohlspiegel 3 und die beiden Teilerspiegel 5 und 6 sind so gross gewählt, dass auch ein binokulares Sehen der Optotypen möglich ist. Gewünschtenfalls kann die Vorrichtung so abgeändert werden, dass der Strahlengang in zwei Teilstrahlengänge zur Prüfung jedes Auges einzeln aufgeteilt wirde.

In dem gezeigten Ausführungsbeispiel ist der Kollimator als Hohlspiegel ausgebildet. Es ist jedoch auch die Verwendung einer Kollimatorlinse möglich, wobei die Optotypen dann in der Brennebene der Linse angeordnet sind.

Als Träger für die Optotypen kann ein handelsüblicher Diaprojektor verwendet werden, wobei dessen Objektiv weggelassen wird. Dadurch kann eine grosse Zahl verschiedener Optotypen mit schnellem Zugriff durch Auswechseln der Dias verwendet werden.

## Patentansprüche

1. Vorrichtung zur Darstellung von Optotypen bei der subjektiven Prüfung von Fehlsichtigkeit, wobei die Optotypen auf die Netzhaut des zu prüfenden Auges mittels eines Kollimators über einen Teilerspiegel im bildseitigen Strahlengang des Kollimators abgebildet werden, mit einem Gehäuse, in dem der Kollimator und die in seiner dingseitigen Brennebene angebrachten Optotypen angeordnet sind, dadurch gekennzeichnet, dass der Teilerspiegel (6) ausserhalb des Gehäuses (8) und so angeordnet ist, dass das zu prüfende Auge am Gehäuse vorbei durch den Teilerspiegel in den freien Raum sieht, und dass das Gehäuse eine Öffnung (14) für den bildseitigen Strahlengang vom Kollimator zum Teilerspiegel aufweist.

2. Vorrichtung nach Anspruch 1, dadurch gekennzeichnet, dass der Teilerspiegel (6) unter 45° geneigt zur optischen Achse des Kollimators (2) angeordnet ist.

3. Vorrichtung nach Anspruch 1 oder 2, dadurch gekennzeichnet, dass der Teilerspiegel (6) und der Kollimator (2) so dimensioniert sind, dass sie binokulares Sehen der Optotypen (1) ermöglichen.

4. Vorrichtung nach einem der Ansprüche 1 bis 3, dadurch gekennzeichnet, dass der Kollimator (2) als Hohlspiegel (3) ausgebildet ist und dass die Optotypen (1) zum Hohlspiegel mittels eines zweiten Teilerspiegels (5) abgebildet werden.

## Claims

1. An apparatus for displaying optotypes during the subjective examination of defective vision, the optotypes being projected into the retina of the eye to be examined by means of a collimator via a divider mirror in the image-side collimator beam path, comprising a housing in which the collimator and the optotypes positioned in the object-side focal plane are located, characterised in that the divider mirror (6) is mounted external to the housing (8) such that the eye to be examined looks past the housing, through the divider mirror into free space, and that the housing has an aperture (14) for the image-side beam path from the collimator to the divider mirror.

2. The apparatus according to claim 1, wherein the divider mirror (6) is mounted at an angle of 45° relative to the optical axis of the collimator (2).

3. The apparatus according to claim 1 or 2, wherein the divider mirror (6) and the collimator (2) are dimensioned such that they make it possible to view the optotypes (1) binocularly.

4. The apparatus according to one of claims 1 to 3, wherein the collimator (2) is designed as a concave mirror (3) and that the optotypes (1) are projected on to the concave mirror by means of a second divider mirror (5).

## Revendications

1. Dispositif pour la présentation de caractères destinés à la correction de la vue (ou «optotypes») lors de l'examen subjectif des défauts de celle-ci, dans lequel on forme une image de ces caractères sur la rétine de l'œil à examiner à l'aide d'un collimateur par l'intermédiaire d'un miroir semi-transparent interposé sur le faisceau lumineux côté image du collimateur, ce dispositif comportant un boîtier dans lequel sont disposés, le collimateur et les caractères placés à son foyer côté objet, caractérisé en ce que le miroir semi-transparent (6) est disposé à l'extérieur du boîtier (8) et de maniière telle que l'œil à examiner regarde à travers lui dans l'espace libre à côté dudit boîtier, et en ce que ce boîtier comporte une ouverture (14) pour le faisceau lumineux côté image du collimateur en direction du miroir semi-transparent.

2. Dispositif suivant la revendication 1, caractérisé en ce que le miroir semi-transparent (6) est disposé à une inclinaison de 45° par rapport à l'axe optique du collimateur (2).

3. Dispositif suivant l'une des revendications 1 et 2, caractérisé en ce que le miroir semi-transparent (6) et le collimateur (2) sont dimensionnés de façon telle qu'ils permettent la vision binoculaire des caractères (1).

4. Dispositif suivant l'une des revendications 1 à 3, caractérisé en ce que le collimateur (2) est établi sous la forme d'un miroir concave, et en ce que l'image des caractères arrive à ce miroir par le moyen d'un second miroir semi-transparent (5).